# EUROPEAN PATENT APPLICATION

(11) **EP 3 459 523 A1**
(43) Date of publication of application: **27.03.2019**
(21) Application number: 17799318.5
(22) Date of filing: 15.05.2017
(51) Int. Cl.: A61K 8/73, A61K 8/02, A61Q 19/00, A61Q 19/10

(54) **MICROSPHERICAL PARTICLES**

(30) Priority: 16.05.2016 JP 2016097599; 27.09.2016 JP 2016188677
(71) Applicant: Nippon Paper Industries Co., Ltd., Tokyo 114-0002 (JP)
(72) Inventor: KOKUFU Yuuki, Tokyo 114-0002 (JP); YAMAGUCHI Sinya, Tokyo 114-0002 (JP); SAJI Kaoru, Tokyo 114-0002 (JP)
(74) Representative: Hoffmann Eitle
(86) International application number: PCT/JP2017/018147
(87) International publication number: WO 2017/199894

(57) **Abstract**

An object of the present invention is to provide a microspherical particle having an excellent massage effect. Another object of the present invention is to provide a microspherical particle that provides an excellent massage effect and cleaning effect when formulated in a cleaning composition and the like.

A microspherical particle is formed of powdered cellulose so that the microspherical particle has an average particle diameter of 50 to 2,000 µm, a sphericity of 0.7 to 1.0, and a dry hardness of 1 to 5,000. A soft type thereof has a dry hardness of 1 to less than 210, and a hard type thereof has a dry hardness of 210 to 5,000. Such microspherical particle is formulated to make a massage composition or a cleaning composition.

## Description

### Technical Field

The present invention relates to a microspherical particle including powdered cellulose.

### Background Art

In the application of a cleaning composition, such as cleaning cream, and cosmetics, a scrubbing agent has been used to improve cleaning performance and a massage effect. The scrubbing agent is preferred in various countries, especially in the United States.

As such a scrubbing agent, an inorganic pigment, such as talc, mica titanium, and kaolin, and a powder of an organic material such as polyethylene are selected and used. In particular, a polyethylene bead is used as the scrubbing agent that is excellent in availability of a material, manufacturability, and a massage effect (Patent literature 1) .

However, such a scrubbing agent cannot be removed once discharged into the sewerage due to its very small size and is easily accumulated in the environment because of a lack of biodegradability. As such, there is rising concern over environmental destruction in rivers, ocean, and the like, thus creating a demand for a more environmentally friendly alternative.

As the scrubbing agent having biodegradability, a granulated product using crystalline cellulose (Patent Literature 2) and a method of granulating a powdery material such as biodegradable starch and an anionic binder and coating the granulated product with divalent or higher-valent cations (Patent Literature 3) have been proposed. Further, as a cleaning agent composition, a cleaning composition using a fiber such as cellulose and a surfactant (Patent Literature 4) has been proposed.

### Citation List

### Patent Literature

Patent Literature 1: Japanese Patent No. 3032531
Patent Literature 2: Japanese Patent Application Laid-Open No. 2003-261436
Patent Literature 3: Japanese Patent Application Laid-Open No. 2000-302630
Patent Literature 4: WO 01/052798 pamphlet

### Summary of Invention

### Technical Problem

However, in Patent Literature 2, a water-soluble binder is used during granulation to prepare a granulated product of the crystalline cellulose. Thus, when the granulated product is added to water-containing cosmetics or the like, the granulated product tends to collapse due to elution of the binder, thereby causing a problem of reduction in the massage effect.

Further, Patent Literature 3 describes that coating of divalent or higher-valent cations after granulation can provide water resistance even if such a water-soluble binder is used. However, since it is in a form of salt, its powdery product is prevented from being uniformly collapsed, thereby causing a problem of reduction in a cleaning effect.

Further, a product in Patent Literature 4 has a poor hardness compared to a case where an inorganic or organic material powder is used, and thus the product has the poor massage effect (a scrubbing feeling).

Thus, an object of the present invention is to provide a microspherical particle having an excellent massage effect.

Further, another object of the present invention is to provide a microspherical particle that provides an excellent massage effect and cleaning effect when formulated in a cleaning composition or the like.

### Solution to Problem

As a result of devoted research, the inventors of the preset invention found a solution to the above mentioned problems by the following means.
[1] A microspherical particle comprising powdered cellulose, the microspherical particle having an average particle diameter of 50 to 2,000 µm, a sphericity of 0.7 to 1.0, and a dry hardness of 1 to 5,000.
[2] The microspherical particle according to the above item [1], wherein the microspherical particle is a granulated product without a binder for binding the powdered cellulose to each other.
[3] The microspherical particle according to the above item [1], wherein the microspherical particle is substantially formed of the powdered cellulose only.
[4] The microspherical particle according to any one of the above items [1] to [3], wherein the dry hardness is 210 to 5,000.
[5] The microspherical particle according to the above item [4], wherein the powdered cellulose has an average particle diameter of 10 to 50 µm and an average polymerization degree of 50 to 750.
[6] The microspherical particle according to any one of the above items [1] to [3], wherein the dry hardness is 1 to less than 210.
[7] The microspherical particle according to the above item [6], wherein the powdered cellulose has an average particle diameter of 10 to 50 µm and an average polymerization degree of 50 to 2,000.
[8] A massage composition, comprising the microspherical particle according to any one of the above items [1] to [7].
[9] A cleaning composition comprising the microspherical particle according to any one of the above items [1] to [7].

### Advantageous Effects of Invention

The present invention can provide the microspherical particle including the powdered cellulose, which has the excellent massage effect.

The present invention can provide the microspherical particle including the powdered cellulose, which has the excellent massage effect and high cleaning effect.

### Description of Embodiments

Hereinafter, the present invention will be described in detail. Note that, unless otherwise specified, the description of "AA to BB%" means "AA% or more to BB% or less."

### (Microspherical particle)

A microspherical particle of the present invention includes powdered cellulose and has an average particle diameter of 50 to 2,000 µm, a sphericity of 0.7 to 1.0, and a dry hardness of 1 to 5,000.

The microspherical particle of the present invention can be obtained by granulating the powdered cellulose described below and be allowed to include a binder and the like within a range of not inhibiting a desired effect.

Examples of the binder described above can include an organic binder, an inorganic binder, and the like, which improve a binding strength between the powdered cellulose.

However, formulating such a binder may cause pollution of waste water and influence collapsibility required for exhibiting the cleaning effect due to the excessive binding strength between the powdered cellulose. On the other hand, the microspherical particle of the present invention can be formed without formulating so called the binder. Thus, one preferred embodiment of the present invention may include performing granulation in which a desired massage feeling can be obtained without including a binder.

That is, the microspherical particle of the present invention may be a granulated product without a binder for binding the powdered cellulose to each other. Further, the microspherical particle of the present invention may be a granulated product substantially formed only of the powdered cellulose described above.

As a method of obtaining the microspherical particle of the present invention, a known granulation method capable of producing a spherical particle by granulating the powdered cellulose can be used. A wet granulation method such as a tumbling granulation method, a tumbling fluidized granulation method, a centrifugal tumbling granulation method, a fluidized bed granulation method, a stirring tumbling granulation method, a spray drying granulation method, an extrusion granulation method, or a melting granulation method is preferable. The tumbling granulation method is more preferable and the centrifugal tumbling granulation method is further preferable to obtain the microspherical particle of the present invention.

In a case of performing such a centrifugal tumbling granulation method, a centrifugal tumbling granulator such as CF-Granulator (manufactured by Freund Corp.) can be used. The rotation number in performing the centrifugal tumbling granulation varies depending on a device in use, but it can be normally set to 100 to 500 rpm.

When the powdered cellulose is charged into the centrifugal tumbling granulator, the powdered cellulose is preferably wetted in advance by adding water or a liquid mainly composed of water, not to be scattered. During the centrifugal tumbling granulation, water or the liquid mainly composed of water is further sprayed on the powdered cellulose. As water or the liquid mainly composed of water, water only or a mixture solution of water and ethanol or the like may be used; however, using only water is preferable to obtain the granulated product having an excellent hardness and specific gravity. During granulation and drying, the granulated product is formed by an interaction such as a hydrogen bond and an intermolecular force formed between the cellulose. Thus, it is speculated that, when a water ratio in an additive/spray liquid is increased within a balance of not inhibiting a drying process after granulation, the interaction between the cellulose increases and enables the formation of the microspherical particle having an excellent specific gravity and hardness.

Spray conditions (a spray amount, time, and frequency) during such granulation vary depending on the rotation number, an amount of the powdered cellulose as a raw material, and the like, and thus cannot be determined generally. However, as an example, the spray conditions can be determined by appropriately adjusting a balance between a slit air rate and the spray liquid after determining the rotation number. For example, the slit air rate can be adjusted within a range of 100 to 400 L/min with respect to 1 kg of the raw material, the spray amount of water can be adjusted within a range of 0.8 to 1.5 kg in total with respect to 1 kg of the raw material, and granulation time can be adjusted within a range of 1 to 4 hours.

Note that, in the present invention, as a method for achieving to fall an average particle diameter of the microspherical particle within a desired range, it is possible to control granulation conditions of the centrifugal tumbling granulator, or to control by subjecting the granulated microspherical particle to a crushing treatment and a classification treatment.

The microspherical particle of the present invention can be classified into two groups in accordance with the hardness. A dry hardness is preferably used as the hardness.

The dry hardness in the present invention refers to a load (g/mm²) required for crushing (breaking) one particle of the microspherical particle. Such a dry hardness was obtained by measuring a peak value of a crushing strength of one microspherical particle using a particle granule hardness meter (product name: GRANO, manufactured by Okada Seiko Co., Ltd.) and calculating an average value of 20 particles.

In a preferred embodiment, the microspherical particle of the present invention has the dry hardness of 210 to 5,000 g/mm² (hereinafter, in the present specification, the microspherical particle having the dry hardness of 210 to less than 5,000 g/mm² may be referred to as a hard-type microspherical particle). The hard-type microspherical particle is suitable for providing a strong massage feeling. The hard-type microspherical particle has the dry hardness of preferably 240 to 4,500 g/mm², more preferably 240 to 4,000 g/mm². When the dry hardness is less than 210 g/mm², the massage feeling as expected from the hard type is hardly obtained. When the dry hardness exceeds 5,000 g/mm², the microspherical particle exhibits little collapsibility and is not suitable for use in a cleaning composition.

In a preferred embodiment, the microspherical particle of the present invention has the dry hardness of 1 to less than 210 g/mm² (hereinafter, in the present specification, the microspherical particle having the dry hardness of 1 to less than 210 g/mm² may be referred to as a soft-type microspherical particle). The soft-type microspherical particle provides a mild massage feeling, not as strong as the one provided by the hard-type microspherical particle described above, but cleaning performance can be easily improved when the soft-type microspherical particle is formulated in a cleaning composition or the like. The soft-type microspherical particle has the dry hardness of more preferably 20 to less than 210 g/mm², further preferably 30 to 200 g/mm². When the dry hardness is less than 1 g/mm², the microspherical particle easily collapses and thus has a high cleaning effect, but hardly provides the massage feeling. When the dry hardness is more than 210 g/mm², the massage effect increases; however, the microspherical particle exhibits low collapsibility as the soft type, thus it becomes more difficult to obtain the cleaning effect.

The average particle diameter shown in the present invention can be obtained, for example, by adding a sample in an amount of 0.2 g to methanol used as a dispersion medium for a measurement using a laser diffraction/scattering particle size distribution measurement device (for example, Microtrac MT3300EX, manufactured by MicrotracBEL Corp.) and measuring a particle diameter at a cumulative volume of 50% as the average particle diameter.

The hard-type microspherical particle has the average particle diameter of preferably 50 to 2,000 µm, more preferably 50 to 1,700 µm, further preferably 100 to 1,500 µm, further preferably 300 to 900 µm. When the average particle diameter is less than 50 µm, the massage feeling is hardly obtained. When the average particle diameter exceeds 2,000 µm, the massage feeling as the scrubbing agent is more likely to deteriorate due to excessive size of the particle.

The soft-type microspherical particle has the average particle diameter of preferably 50 to 2,000 µm, more preferably 50 to 1,700 µm, further preferably 100 to 1,500 µm, further preferably 200 to 900 µm. When the average particle diameter is less than 50 µm, the massage feeling is hardly obtained. When the average particle diameter exceeds 2,000 µm, the massage feeling as the scrubbing agent is more likely to deteriorate due to excessive size of the particle.

The sphericity as used in the present invention can be obtained by acquiring image data of the microspherical particle as an observation object using an optical microscope (product name: digital microscope VHX-600, manufactured by Keyence Corp.) and then conducting an image analysis with respect to the microspherical particle in the obtained image data by using Image Hyper II (manufactured by Digimo Co., Ltd.). Such a sphericity can be obtained from a formula: sphericity = A/B, where A is an area of the microspherical particle obtained by the image analysis, and B is an area of an imaginary perfect sphere with the diameter equal to the maximum major axis diameter of the microspherical particle, which is obtained by calculation. Thus, the microspherical particle has a shape closer to that of a perfect sphere as the sphericity approaches 1. Conversely, the microspherical particle has a more irregular shape as the sphericity becomes farther from 1. Note that the sphericity is shown as an average value of 20 microspherical particles observed.

The hard-type microspherical particle has the sphericity of preferably 0.7 to 1.0, more preferably 0.8 to 1.0, and further preferably 0.84 to 1.0. As described above, the microspherical particle of the present invention is obtained by granulating the powdered cellulose or the powdered cellulose composition. Accordingly, when the sphericity of such a microspherical particle is less than 0.7, the microspherical particle having a distorted shape tends to collapse from a distorted site as a starting point during massage, thereby making it difficult to continuously obtain the massage feeling, and thus tends to be hardly suitable for the scrubbing agent.

The soft-type microspherical particle has the sphericity of preferably 0.7 to 1.0, and more preferably 0.7 to 0.84. When the sphericity is less than 0.7, the microspherical particle having a distorted shape provides the massage feeling with a gritty sensation and causes too much stimulation feeling to the skin as the soft type, thus being not suitable for the scrubbing agent expected to provide a soft stimulation feeling. Further, when the sphericity is within a range between 0.7 and 0.84, the microspherical particle exhibits some surface roughness while maintaining the sphericity, thereby improving the massage feeling in a low hardness region having the dry hardness of less than 210 g.

The microspherical particle of the present invention can be granulated by including an additive, such as a perfume, a disintegration aid, and a granulation accelerating agent, within a range of not inhibiting the desired effect.

### (Powdered cellulose)

In the present invention, examples of a raw material of the powdered cellulose include, though not particularly limited to, pulp from a broadleaf tree, pulp from a coniferous tree, pulp from a linter, non-wood pulp, and the like. Preferred is to obtain the the powdered cellulose having the small average particle diameter from the viewpoint of convenience in adjusting the granulation of the microspherical particle, and the broadleaf tree pulp having a smaller fiber diameter and fiber width than those of the coniferous tree pulp is preferably used.

Further, in the present invention, examples of a pulping method (a cooking method) include, though not particularly limited to, a sulfite cooking method, a kraft cooking method, a soda-quinone cooking method, an organosolv cooking method, and the like. Of these, the sulfite cooking method causing a low average polymerization degree is preferable from the viewpoint of environmental aspects.

The powdered cellulose used in the present invention can be obtained by crushing the pulp that has been subjected to an acid hydrolysis treatment with a mineral acid such as hydrochloric acid, sulfuric acid, and nitric acid, or by mechanically crushing the pulp that has not been subjected to an acid hydrolysis treatment.

In a case where the powdered cellulose is obtained by subjecting the pulp raw material described above to the acid hydrolysis treatment and the machine crushing, the powdered cellulose is produced through a raw material pulp slurry preparation step, an acid hydrolysis reaction step, a neutralization / washing / liquid removal step, a drying step, a crushing step, and a classification step.

The pulp raw material can be in a flowable state or in a sheet shape. In a case where flowable pulp from a pulp-bleaching step is used as a raw material, a concentration of the pulp raw material needs to be increased before charging the pulp raw material into a hydrolysis reaction tank. Thus, the pulp raw material is concentrated by a dehydrator such as a screw press and a belt filter and a predetermined amount of the pulp raw material is charged into the reaction tank. In a case where a dry sheet of the pulp is used as a raw material, the pulp is loosened by a crusher such as a roll crusher or the like and then charged into the reaction tank.

Next, a dispersion having a pulp concentration of 3 to 10% by weight (in terms of solid content), which has been adjusted to have an acid concentration of 0.10 to 1.2 N, is treated under conditions of a temperature of 80 to 100°C and a duration of 30 minutes to 3 hours. After the hydrolysis treatment of the pulp, a solid-liquid separation is performed to separate into the hydrolyzed pulp and the waste acid in the dehydration step. The hydrolyzed pulp is neutralized by adding an alkaline agent and washed. Subsequently, the washed product is dried in a dryer, and then mechanically crushed and classified by a crusher into a predetermined size.

Examples of the crusher include: a cutting type mill such as a mesh mill (manufactured by Horai Co., Ltd.), ATOMS (manufactured by K. K. Yamamoto Hyakuma Seisakusho), a knife mill (manufactured by Pallmann Industries, Inc.), a cutter mill (manufactured by Tokyo Atomizer M.F.G. Co., Ltd.), CS cutter (manufactured by Mitsui Mining Co., Ltd.), a rotary cutter mill (manufactured by Nara Machinery Co., Ltd.), a pulp coarse crusher (manufactured by Zuiko Co., Ltd.), and a shredder (manufactured by Shinko-Pantec Co., Ltd); a hammer type mill such as a jaw crusher (manufactured by Makino Corp.) and a hammer crusher (manufactured by Makino Mfg. Co., Ltd.); an impact mill such as a pulverizer (manufactured by Hosokawa Micron Corp.), Fine Impact Mill (manufactured by Hosokawa Micron Corp.), Super Micron Mill (manufactured by Hosokawa Micron Corp.), Inomizer (manufactured by Hosokawa Micron Corp.), Fine Mill (manufactured by Nippon Pneumatic Mfg. Co., Ltd.), a centrifugal mill (CUM model) (manufactured by Mitsui Mining Co., Ltd.), Exceed Mill (manufactured by Makino Mfg. Co., Ltd.), Ultraplex (manufactured by Makino Mfg. Co., Ltd.), Contraplex (manufactured by Makino Mfg. Co., Ltd.), Kolloplex (manufactured by Makino Mfg. Co., Ltd.), a sample mill (manufactured by Seishin Enterprise Co., Ltd.), a bantam mill (manufactured by Seishin Enterprise Co., Ltd.), an atomizer (manufactured by Seishin Enterprise Co., Ltd.), a tornado mill (manufactured by Nikkiso Co., Ltd.), NEA Mill (manufactured by Dalton Corp.), a fine pulverizer (HT model) (manufactured by Horai Co., Ltd.), Jiyu Mill (manufactured by Nara Machinery Co., Ltd.), New Cosmomizer (manufactured by Nara Machinery Co., Ltd.), a gather mill (manufactured by Nishimura Machine Works Co., Ltd.), Super Powder Mill (manufactured by Nishimura Machine Works Co., Ltd.), Blade Mill (manufactured by Nisshin Engineering Inc.), Super Rotor (manufactured by Nisshin Engineering Inc.), an NPa crusher (manufactured by Sansho Industry Co., Ltd.), a Wiley mill (manufactured by K.K. Miki Seisakusho), a pulp mill (Zuiko Co., Ltd.), Jacobson Mill (manufactured by Shinko-Pantec Co., Ltd.), and a universal mill (manufactured by Tokuju Co., Ltd.); an airflow mill such as a CGS-type jet mill (manufactured by Mitsui Mining Co., Ltd.), Micron Jet (manufactured by Hosokawa Micron Corp.), Counter Jet Mill (manufactured by Hosokawa Micron Corp.), Cross Jet Mill (manufactured by Kurimoto, Ltd.), Supersonic Jet Mill (manufactured by Nippon Pneumatic Mfg. Co., Ltd.), Current Jet (manufactured by Nisshin Engineering Inc.), a jet mill (manufactured by Sansho Industry Co., Ltd.), EBARA Jet Micronizer (manufactured by Ebara Corp.), Ebara Triad Jet (manufactured by Ebara Corp.), Ceren Miller (manufactured by Masuko Sangyo Co., Ltd.), New Microcyclomat (manufactured by Masuno Seisakusho Ltd.), and Kryptron (manufactured by Kawasaki Heavy Industries, Ltd.); and a vertical roller mill such as a vertical roller mill (manufactured by Scenion Inc.), a vertical roller mill (manufactured by Schaeffler Japan Co., Ltd.), a roller mill (manufactured by Kotobuki Engineering & Manufacturing Co., Ltd.), VX Mill (manufactured by Kurimoto, Ltd.), KVM Vertical Mill (manufactured by Earthtechnica Co, Ltd.), and IS Mill (manufactured by IHI Plant Engineering Corp.) .

For the purpose of imparting a function to or improving a function of the powdered cellulose of the present invention, the raw material of the powdered cellulose can be mixed with one or two or more other organic and/or inorganic components in an arbitrary ratio, and crushed. Further, a chemical treatment may be applied within a range that does not significantly impair a polymerization degree of natural cellulose used as the raw material.

On the other hand, in a case where the powder is produced only by machine crushing using the pulp that has not been subjected to the acid hydrolysis treatment as a raw material, a vertical roller mill having high fine crushability is preferably used as the crusher. In the present invention, the vertical roller mill refers to a centrifugal vertical crusher belonging to roller mills and performs crushing by grinding the raw material with a discoid turn table and a vertical roller. The most distinctive feature of the vertical roller mill is its excellent fine crushability and, as a reason for this, it can be mentioned that the raw material is crushed by a force to compress the raw material between the roller and the table and a shearing force generated between the roller and the table. Examples of the crusher conventionally used include a vertical roller mill (manufactured by Scenion Inc.), a vertical roller mill (manufactured by Schaeffler Japan Co., Ltd.), a roller mill (manufactured by Kotobuki Engineering & Manufacturing Co., Ltd.), VX Mill (manufactured by Kurimoto, Ltd.), KVM Vertical Mill (manufactured by Earthtechnica Co, Ltd.), IS Mill (manufactured by IHI Plant Engineering Corp.), and the like.

It is preferable that the powdered cellulose used for the hard-type microspherical particle has the average particle diameter of 10 to 50 µm and the average polymerization degree of 50 to 750.

It is preferable that the powdered cellulose used for the soft-type microspherical particle has the average particle diameter of 10 to 50 µm and the average polymerization degree of 50 to 2,000.

The powdered cellulose used for the hard-type microspherical particle has the average particle diameter of preferably 10 to 50 µm, more preferably 15 to 40 µm. When the average particle diameter of the powdered cellulose is less than 10 µm, it becomes difficult to granulate the microspherical particle due to its small particle size. Further, when the average particle diameter of the powdered cellulose exceeds 50 µm, it becomes difficult to perform the granulation due to its large particle size.

The powdered cellulose used for the soft-type microspherical particle has the average particle diameter of preferably 10 to 50 µm, more preferably 15 to 40 µm. When the average particle diameter of the powdered cellulose is less than 10 µm, it becomes difficult to granulate the microspherical particle due to its small particle size. Further, when the average particle diameter of the powdered cellulose exceeds 50 µm, it becomes difficult to perform the granulation due to its large particle size.

The powdered cellulose used for the hard-type microspherical particle has the average polymerization degree of preferably 50 to 750, more preferably in a range of 100 to 500. When the average polymerization degree is higher than the above range, a strength of the powdered cellulose itself becomes high. Thus, the powdered cellulose is hardly compressed during the granulation and the microspherical particle becomes bulky. As a result, the dry hardness tends to be insufficient as the hard-type microspherical particle. On the other hand, when the average polymerization degree is lower than the above range, cellulose fibers have less entanglement during the granulation. Thus, the dry hardness of the microspherical particle tends to deteriorate.

The powdered cellulose used for the soft-type microspherical particle has the average polymerization degree of preferably 50 to 2,000, more preferably in a range of 100 to 1,500. When the average polymerization degree is higher than the above range, a strength of the powdered cellulose itself becomes high. Thus, the powdered cellulose is hardly compressed during the granulation and the microspherical particle becomes bulky. As a result, the dry hardness becomes insufficient as the soft-type microspherical particle. On the other hand, when the average polymerization degree is lower than the above range, cellulose fibers have less entanglement during the granulation. Thus, the dry hardness of the microspherical particle tends to deteriorate.

The microspherical particle of the present invention has the excellent massage effect and cleaning effect presumably by the following reasons. That is, as the average particle diameter becomes larger, a contact area with the skin increases, thereby enhancing the massage feeling. However, when the average particle diameter of the microspherical particle using conventional cellulose becomes larger, the more binder needs to be formulated for the granulation. Thus, it is considered that this formulation likely causes deformation or the like during the granulation, resulting in impairment of the massage feeling as well as a reduction in the collapsibility leading to deterioration of the cleaning effect. It is speculated that the microspherical particle of the present invention, which maintains the sphericity and the dry hardness within the predetermined ranges, does not require the binder regardless of the average particle diameter and thus can simultaneously achieve both the massage effect and the cleaning effect.

The microspherical particle of the present invention may be used for massaging by directly applying an aggregate of the microspherical particle to the skin or used as a massage composition by mixing with a base. Any base can be used for the massage composition without a particular limitation as long as it serves as a medium for dispersing the microspherical particle of the present invention and is applicable to the skin.

Further, a composition including the microspherical particle of the present invention can be used as a cosmetic composition.

The microspherical particle of the present invention can be used as a cleaning composition together with a cleaning component having foamability such as a body soap and a hand soap. These compositions may be those containing a surface active substance, as a main agent, such as fatty acid sodium, fatty acid potassium, alpha-sulfo fatty acid ester sodium, sodium linear-alkylbenzene sulfonate, sodium alkyl sulfate, sodium alkylether sulfate, sodium alpha-olefin sulfonate, sodium alkyl sulfonate, sucrose fatty acid ester, sorbitan fatty acid ester, polyoxyethylene sorbitan fatty acid ester, fatty acid alkanol amide, polyoxyethylene alkyl ether, polyoxyethylene alkyl phenyl ether, alkylamino fatty acid sodium salt, alkyl betaine, alkyl amine oxide, alkyl trimethyl ammonium salt, dialkyl dimethyl ammonium salt, or the like. Further, an auxiliary agent may be sodium carbonate, sodium silicate, zeolite, citric acid and its salt, EDTA (ethylenediaminetetraacetic acid) and its salt, hydroxyethane phosphonic acid, L-aspartic acid diacetic acid (ASDA), L-glutamic acid diacetic acid (GLDA), sodium sulfate, or the like. Furthermore, as necessary, they can contain glycerol and polyethylene glycol, a thickener, a perfume, water, ethanol or the like.

The microspherical particle of the present invention includes the chemically stable powdered cellulose as a main component and thus can be a component of the cleaning composition without inhibiting an action of the cleaning component described above. As a result, a high cleaning effect can be obtained by the cleaning component and the microspherical particle. Furthermore, since the microspherical particle provides the excellent massage feeling, the composition is excellent as the massage composition and also can be used as the cosmetic composition.

### Examples

The present invention will be described in detail below by way of examples; however, the present invention is not limited by the following examples.

### <Example 1H>

Powdered cellulose W-100G (manufactured by Nippon Paper Industries Co., Ltd., average particle diameter of 35 µm, average polymerization degree of 450, apparent specific gravity of 0.29 g/ml, angle of repose of 58°) in an amount of 0.5 kg was charged into a centrifugal tumbling granulator CF-360N (manufactured by Freund Corp.) and granulation was performed by spraying water in an amount of 1.25 kg in 100 minutes in a slit air rate of 200 to 300 L/min while a rotary disk is rotated. The generated particle was fluidized and dried, thus obtaining a microspherical particle having an average particle diameter of 650 µm, a sphericity of 0.85, a dry hardness of 452 g, and an apparent specific gravity of 0.65 g/ml.

### <Example 2H>

Powdered cellulose W-400G (manufactured by Nippon Paper Industries Co., Ltd., average polymerization degree of 150, average particle diameter of 24 µm, apparent specific gravity of 0.48 g/ml, angle of repose of 52°) in an amount of 1 kg was charged into a centrifugal tumbling granulator CF-360N (manufactured by Freund Corp.) and granulation was performed by spraying water in an amount of 1.2 kg in 100 minutes in a slit air rate of 220 L/min while a rotary disk is rotated. The generated particle was fluidized and dried, thus obtaining a microspherical particle having an average particle diameter of 340 µm, a sphericity of 0.84, a dry hardness of 247 g, and an apparent specific gravity of 0.83 g/ml.

### <Example 3H>

A microspherical particle having an average particle diameter of 490 µm, a sphericity of 0.87, a dry hardness of 490 g, and an apparent specific gravity of 0.85 g/ml was obtained in the same manner as that in Example 2H except that the number of spraying times of water was increased during 100 minutes of the granulation.

### <Example 1S>

Powdered cellulose W-100GK (manufactured by Nippon Paper Industries Co., Ltd., average particle diameter of 37 µm, average polymerization degree of 1420, apparent specific gravity of 0.32 g/ml) in an amount of 1.0 kg was charged into a centrifugal tumbling granulator CF-360N (manufactured by Freund Corp.) and granulation was performed by spraying water in an amount of 1.2 kg in 100 minutes in a slit air rate of 200 L/min while a rotary disk is rotated. The generated particle was fluidized and dried, thus obtaining a microspherical particle having an average particle diameter of 547 µm, a sphericity of 0.73, a dry hardness of 39 g, and an apparent specific gravity of 0.38 g/ml.

### <Example 2S>

Powdered cellulose W-400M (manufactured by Nippon Paper Industries Co., Ltd., average particle diameter of 24 µm, average polymerization degree of 130, apparent specific gravity of 0.48 g/ml) in an amount of 1.0 kg was charged into a centrifugal tumbling granulator CF-360N (manufactured by Freund Corp.) and granulation was performed by spraying water in an amount of 1.2 kg in 100 minutes in a slit air rate of 220 L/min while a rotary disk is rotated. The generated particle was fluidized and dried, thus obtaining a microspherical particle having an average particle diameter of 440 µm, a sphericity of 0.78, a dry hardness of 148 g, and an apparent specific gravity of 0.74 g/ml.

### <Example 3S>

A microspherical particle having an average particle diameter of 211 µm, a sphericity of 0.79, a dry hardness of 180 g, and an apparent specific gravity of 0.80 g/ml was obtained in the same manner as that in Example 2S except that the number of spraying times of water was increased during 100 minutes of the granulation.

### <Reference example>

A polyethylene bead (product name: Microscrub 35PC, manufactured by Prospector Corp.) having an average particle diameter of 350 µm and a sphericity of 0.38 was used instead of the microspherical particle including the powdered cellulose.

### <Evaluation>

### <Average particle diameter>

A laser diffraction/scattering particle size distribution measurement device (Microtrac MT3300EX, manufactured by MicrotracBEL Corp.) was used. A measurement was performed with a sample in an amount of 0.2 g, which was added to methanol used as a dispersion medium in the measurement, thus obtaining a particle diameter at a cumulative volume of 50% (the average particle diameter).

### <Measurement of sphericity>

Image data of the microspherical particle as an observation object was acquired using an optical microscope (product name: digital microscope VHX-600, manufactured by Keyence Corp.) and image analysis was performed using Image Hyper II (manufactured by Digimo Co., Ltd.). The sphericity was obtained from the formula: sphericity = A/B, where A was an area of the microspherical particle obtained by the image analysis, and B was an area of an imaginary perfect sphere obtained by calculation, the diameter of which is the maximum major axis diameter of the microspherical particle.

### <Measurement of dry hardness>

A peak value of a crushing strength of one microspherical particle was measured using a particle granule hardness meter (product name: GRANO, manufactured by Okada Seiko Co., Ltd.) and an average value of 20 particles was obtained as the dry hardness (g).

### <Evaluation of massage (body)>

To 95 g of a commercially available body cleanser (product name: Dove body wash G, manufactured by Unilever Japan K.K.), 5 g of the microspherical particles of each of Examples 1H to 3H and 1S to 3S or the polyethylene beads in Reference example were added and mixed well to produce a mixture liquid. After the mixture liquids thus obtained were each left to stand for 5 hours, 5 g of each mixture liquid was applied to the cheeks of 5 subjects and then the applied part was rubbed 20 times by the palm. Tactile sensation that the subject had when rubbing was evaluated and represented as an average value. Note that final evaluation was classified into the following categories A to D.
A: Excellent tactile sensation with strong massage feeling
B: Ordinary tactile sensation with ordinary massage feeling
C: Ordinary tactile sensation with poor massage feeling
D: No tactile sensation with no massage feeling

Results of Examples 1H to 3H and Reference example are shown in Table 1. Further, results of Examples 1S to 3S and Reference example are shown in Table 2.

### <Massage effect (scalp)>

To 95 g of a commercially available shampoo (product name: Merit, manufactured by Kao Corp.), 5 g of the microspherical particles of each of Examples 1H to 3H and 1S to 3S or the polyethylene beads in Reference example were added and mixed well to produce a mixture liquid. After the mixture liquids thus obtained were each left standing for 5 hours, 0.5 g of each mixture liquid was applied to the scalps of 5 subjects and then the applied part was rubbed 10 times by the fingers. Tactile sensation that the subject had when rubbing was evaluated and represented as an average value.
A: Excellent tactile sensation with strong massage feeling
B: Ordinary tactile sensation with ordinary massage feeling
C: Ordinary tactile sensation with poor massage feeling
D: No tactile sensation with no massage feeling

Results of Examples 1H to 3H and Reference example are shown in Table 1. Further, results of Examples 1S to 3S and Reference example are shown in Table 2.

### <Massage effect (inside mouth)>

To 95 g of a commercially available toothpaste (product name: Guard hello standing tube, manufactured by Kao Corp.), 5 g of the microspherical particles of each of Examples 1H to 3H and 1S to 3S or the polyethylene beads in Reference example were added and mixed well to produce a mixture liquid. After the mixture liquids thus obtained were each left standing for 5 hours, 1 g of each mixture liquid was taken up by the fingers of 5 subjects and then applied inside the mouth and on the gums by rubbing 10 times. Tactile sensation that the subject had when rubbing was evaluated and represented as an average value.
A: Excellent tactile sensation with strong massage feeling
B: Ordinary tactile sensation with ordinary massage feeling
C: Ordinary tactile sensation with poor massage feeling
D: No tactile sensation with no massage feeling

Results of Examples 1H to 3H and Reference example are shown in Table 1. Further, results of Examples 1S to 3S and Reference example are shown in Table 2.

### <Evaluation of cleaning performance>

To 95 g of a commercially available body soap (product name: Biore uRf, manufactured by Kao Corp.), 5 g of the microspherical particles of each of Examples 1S to 3S or the polyethylene beads in Reference example were added to produce a cleaning liquid. An area measuring 2 × 2 cm on the left palm of each panelist was uniformly painted with a blue oily marking pen (Hi-Mackee Care, manufactured by Zebra Co., Ltd.). Subsequently, 5 g of each of the above cleaning liquids was applied to the painted part to clean by rubbing 100 times with both palms. After washed with water and dried, the palm was observed with 20x magnification using a microscope (VH-7000, manufactured by Keyence Corp.) to evaluate a removal degree (the cleaning performance) of the blue marking. Results are shown in Table 2.
A: Excellent cleaning performance with blue marking nearly completely removed
B: Ordinary cleaning performance with blue marking mostly removed
C: Poor cleaning performance with blue marking not removed very well
D: No cleaning performance with blue marking not removed at all

### [Table 1]

**Table 1 (Hard Type)**

| | Raw Material of Microspherical Particle | Microspherical Particle | | | Massage Effect | Massage Effect | Massage Effect |
|---|---|---|---|---|---|---|---|
| | | Average Particle Diameter | Sphericity | Dry Hardness | Body | Scalp | Inside Mouth |
| | | (µm) | | | | | |
| Example 1H | W-100G | 650 | 0.85 | 452 | A | A | A |
| Example 2H | W-400G | 340 | 0.84 | 247 | A | A | A |
| Example 3H | W-400G | 490 | 0.87 | 490 | A | A | A |
| Reference Example | polyethylene beads | 350 | 0.38 | - | A | A | A |

### [Table 2]

**Table 2 (Soft Type)**

| | Raw Material of Microspherical Particle | Microspherical Particle | | | Massage Effect | Massage Effect | Massage Effect | Evaluation of Cleaning Performance |
|---|---|---|---|---|---|---|---|---|
| | | Average Particle Diameter | Sphericity | Dry Hardness | Body | Scalp | Inside Mouth | |
| | | (µm) | | | | | | |
| Example 1S | W-100GK | 547 | 0.73 | 39 | B | B | B | A |
| Example 2S | W-400M | 440 | 0.78 | 148 | B | B | B | A |
| Example 3S | W-400M | 211 | 0.79 | 180 | B | B | B | B |
| Reference Example | polyethylene beads | 350 | 0.38 | - | A | A | A | D |

## Claims

1. A microspherical particle comprising powdered cellulose, the microspherical particle having an average particle diameter of 50 to 2,000 µm, a sphericity of 0.7 to 1.0, and a dry hardness of 1 to 5,000.

2. The microspherical particle according to claim 1, wherein the microspherical particle is a granulated product without a binder for binding the powdered cellulose to each other.

3. The microspherical particle according to claim 1, wherein the microspherical particle is substantially formed of the powdered cellulose only.

4. The microspherical particle according to any one of claims 1 to 3, wherein the dry hardness is 210 to 5,000.

5. The microspherical particle according to claim 4, wherein the powdered cellulose has an average particle diameter of 10 to 50 µm and an average polymerization degree of 50 to 750.

6. The microspherical particle according to any one of claims 1 to 3, wherein the dry hardness is 1 to less than 210.

7. The microspherical particle according to claim 6, wherein the powdered cellulose has an average particle diameter of 10 to 50 µm and an average polymerization degree of 50 to 2,000.

8. A massage composition, comprising the microspherical particle according to any one of claims 1 to 7.

9. A cleaning composition comprising the microspherical particle according to any one of claims 1 to 7.
